# EUROPEAN PATENT APPLICATION

(11) **EP 4 468 302 A1**
(43) Date of publication of application: **27.11.2024**
(21) Application number: 24177035.3
(22) Date of filing: 21.05.2024
(51) Int. Cl.: G16H 20/13, G16H 40/63, G16H 40/67, G16H 50/20

(54) **COMPUTER-IMPLEMENTED METHOD FOR MONITORING THE INHALATION OF A DRUG, SYSTEM INCLUDING INHALATION DEVICE, COMPUTER PROGRAM, AND STORAGE MEDIUM ASSOCIATED THEREOF**

(30) Priority: 22.05.2023 PT 2023118671
(71) Applicant: Ebreathie - Breathing Solutions, Lda., 2735-544 Agualva-Cacém (PT)
(72) Inventor: RAIMUNDO, Ana Rita Batista Constante, 2735-544 Agualva-Cacém (PT); REBELO, Ana Filipa Oliva, 2735-544 Agualva-Cacém (PT); REBELO, Ana Rita Oliva, 2735-544 Agualva-Cacém (PT); COSTA, Alexandre Miguel Manta Da, 2735-544 Agualva-Cacém (PT)
(74) Representative: Monteiro Alves, Inês

(57) **Abstract**

An inhalation monitoring device (10) connected to a therapeutic pump (2) comprises differential pressure sensors that measure a patient's airflow when inhaling a drug for the treatment of a respiratory disease. The resulting data are processed by a first computing unit of the inhalation monitoring device (10) and a light is emitted from a light source (16) located in the casing (17), indicating the inhalation quality. The inhalation monitoring device (10) sends the data to a second computing unit (20) that processes it, displaying the results and data in a user interface (21). The second computing unit (20) sends the processed data to a third computing unit (30) for database storage and further training of an artificial intelligence model that predicts the progression of acute respiratory disease and identifies potential flare-up triggers.

## Description

### TECHNICAL DOMAIN

The subject matter addressed by this application is encompassed by computer-implemented inventions for monitoring drug inhalation. In particular, this application relates to a computer-implemented method for monitoring drug inhalation quality and a system comprising a drug inhalation monitoring device and the implementation of this method. This application also addresses computer programs with logic instructions for implementing the method defined in this application and their respective computer-readable data storage media.

### BACKGROUND

Asthma is a disease that affects the lungs and can cause symptoms such as coughing, shortness of breath, wheezing, and chest tightness. According to the World Health Organization (WHO), asthma affects about 235 million people worldwide, and is one of the leading causes of chronic disease in children.

Although it is a common disease, the current global asthma landscape varies greatly by region and country. In general, the prevalence of asthma is higher in developed countries than in developing nations. Moreover, asthma affects children more than adults, although this disease may develop at any age.

In recent years, there has been a growing awareness of asthma and its symptoms, as well as the importance of early diagnosis and appropriate treatment. New therapies are under development for improving asthma control and reducing the frequency and severity of its symptoms.

However, despite these advances, asthma continues to pose a major public health challenge worldwide. Lack of access to proper diagnoses and treatments, as well as exposure to environmental pollutants and other risk factors, are still major problems that must be addressed to improve disease control.

### PRIOR ART

Several asthma treatments are currently available, designed to ease airway inflammation and relieve the symptoms of this disease. Most of these treatments focus on dilating airways in order to allow air to flow more freely, with patients experiencing normal pulmonary function s. In such treatments, a therapeutic pump is typically used that, when pressure is applied by a patient or caregiver, emits a spray of medication contained therein under pressure that enters the patient's lungs through inhalation. In this process, it is extremely important for the patient to have a good inhalation technique, so that the drug can pass through the upper airways and be deposited on the pulmonary alveoli, where the treatment takes effect. However, it often happens that patients or caregivers do not have good inhalation techniques, whereby the patient does not perform the inhalation within the drug release time window, or does not inhale the amount of air needed to carry the powder drug to the lungs. In these situations, the treatment proves to be almost or even completely inefficacious.

US Patent Application N° 2016/0144141 A1, filed by Cognita Labs LLC, Houston, TX, USA, and published on May 26, 2016, discloses a detachable cap for measuring the usage of an inhaler that includes a hollow receiving portion adapted to removably receive the inhaler. A vent is formed in an upper portion of the cap allowing air to flow through the cap to the inhaler. This cap includes a pressure sensor that measures the differential pressure v alongside the air inflow vent. This patent application does not address the inclusion of data on weather conditions and airborne allergen concentrations in the patient's geographic area; neither does it consider individual patient characteristics in its airflow calculations.

There is thus still no technological solution at the state of the art that ensures exact and precise inhalation quality monitoring during the treatment of asthma patients, as current solutions cannot monitor inhalation quality or efficacy, instead monitoring only the time when the inhalations took place. Inhalation effectiveness is one of the key factors for assessing whether the asthma treatments are being administered correctly, in order to reach correct conclusions about adherence to the treatment of the disease.

### SUMMARY OF THE INVENTION

This invention relates to a computer-implemented method for monitoring the quality of inhalations performed by a patient with a therapeutic pump containing a drug, wherein the therapeutic pump is connected to an inhalation monitoring device that comprises differential pressure sensors that are configured to collect data relating to inhalations performed by the patient during the inhalation of the drug contained in the therapeutic pump. This invention also relates to a system that implements the method and that comprises, moreover to the said inhalation monitoring device, a second computing unit which could be the smartphone of the patient performing the inhalation, for example, and a third computing unit that could be, for example, a server that stores inhalation data. In certain embodiments of this invention, the third computing unit performs an artificial intelligence model training step.

In a first aspect, this invention relates to a computer-implemented method for monitoring the quality of inhalations performed by a patient with a therapeutic pump containing a drug, wherein the therapeutic pump is connected to an inhalation monitoring device.

In a second aspect, this invention relates to a system configured to implement the method defined in the first aspect of the invention, which includes a first computing unit, a second computing unit, and a third computing unit.

In a third aspect, this invention relates to computer program products that comprise logic instructions for making the first computing unit, the second computing unit and /or the third computing unit perform the steps of the method described in the first aspect of this invention.

In a fourth aspect, this invention relates to a computer-readable storage medium that comprises the installation of a computer program product as defined in the third aspect of the invention.

### SOLUTION TO THE PROBLEM

The technological solution provided by the computer-implemented method and system defined by this invention allows monitoring the inhalation quality of a patient with asthma on some other respiratory disease who is undergoing drug inhalation treatment with a therapeutic pump. This invention relates to a computer-implemented method and system comprising an inhalation monitoring device, wherein this inhalation monitoring device is connected to a conventional inhaler or therapeutic pump, whereby the inhalation monitoring device collects inhalation data through one or more built-in pressure sensors, providing patients with feedback on their inhalation quality. This information is provided by a LED light located in the inhalation monitoring device casing, which lights up in a specific color (such as green) if the inhalation was successful, and in another color (such as red) if the inhalation was not effective. In certain embodiments of this invention, the inhalation monitoring device additionally comprises one or more vibration and motion sensors, such as one or more accelerometers, configured to detect incorrect positioning of the inhaler and consequently the inhalation monitoring device as well during inhalation by the patient. This incorrect positioning data is sent to the second computing unit, where it is processed for inclusion in the patient's statistics. Another example of using vibration and motion sensors is to emit vibrations, for example, if the patient, their caregiver, or any aid provider, needs to locate the inhaler. Through the second computing unit, the user can issue a vibrate order for locating the inhalation monitoring device. Decisions made on the basis of data collected by the sensors are supplemented by user-entered patient data on a device, such as a dedicated app on the patient's smartphone, with such data then used in further processing through the method addressed by this invention. Furthermore, in preferred embodiments of this invention, air quality data for the geographical area where the patient is located may be obtained through the method and system addressed by this invention, whereby they may be taken into consideration during processing and making decisions about adherence to the treatment. Inhalation efficacy consists of defining whether the drug has managed to flow deep into the patient's bronchi and achieved the desired upper lung deposition. In technical terms, this efficacy consists of assessing whether the drug was released into the inhaler within the optimal window of an inhalation cycle. This point will be discussed below. This evaluation is made by correlating the inhaled airflow and the plunger pressure timing in the therapeutic pump.

The data collected on each inhalation by a patient, as well as the metrics resulting from the processing thereof, are stored for comparison with data on subsequent inhalations. This long-term monitoring underpins assessments of the patient's pulmonary function throughout their treatment.

In terms of comparing inhalation profiles with benchmark models, thresholds have been established for patient profiles, such as their age, weight, and height. These benchmark thresholds will be taken into account in the comparisons used while processing the data.

Furthermore, the method and system according to this invention make it possible to train a machine learning artificial intelligence model that will use the large amount of data collected from the plurality of inhalations and the other data introduced, as described in this patent application, to identify patterns and provide information on potential triggers for asthma attacks, such as pollen, dust, air humidity, etc., or more severe predictions for the therapeutic condition that can cause uncontrolled disease and potential asthma attacks.

The technological solution presented by this invention, more particularly the inhalation monitoring device, is configured to store data collected by the sensors and then process them in order to calculate the metrics needed to define the effectiveness of the inhalation, well also storing the results of these calculations. When the inhalation monitoring device is in communication by a wired or wireless means, such as bluetooth, with another computing unit, such as the patient's smartphone, the data and calculation results are sent to the smartphone, which in turn sends them to a server or a third computing unit, whereby they will be stored in a database and processed further by the server or the third computing unit.

In brief, the technological solution presented by this invention allows a patient with a respiratory condition such as asthma, whose treatment includes inhaling medication, to optimize their inhalation technique and obtain immediate information about each inhalation, while also obtaining information about their pulmonary function over time.

### ADVANTAGEOUS EFFECTS OF THE INVENTION

The technological solution presented by this invention has several advantages over the previous state of the art. For example, the system and method according to this invention may be used in two modes: a tutorial mode and an inhalation monitoring mode.

This means that patients will be able to use their inhalation monitoring devices connected to a therapeutic pump just to train their inhalation technique, regardless of whether or not any drugs are released by the inhaler. This training will keep the patient informed about any faults that may be corrected during inhalation, such as the inhaler position, the best time to release the drug, the best time to inhale, the amount of air to inhale, etc.

Once the patient improves their inhalation technique, the inhalation monitoring device connected to a therapeutic pump is used in a normal manner, with more steps added to their inhalation technique, in addition to the inhalation monitoring device being connected to the therapeutic pump. At the end of each inhalation, the patient receives information from an indicator light, such as a Light Emitting Diode (LED) light that tells him about the inhalation quality. The patient will also be able to find out more about their inhalation quality and the progress of their disease by consulting an app on a mobile device, for example, with data updated to the last inhalation.

Moreover, the inhalation monitoring device according to this invention is connected to the therapeutic pump in a manner that does not obstruct the airflow, allowing pressure sensors that are advantageously placed on the surface facing the airflow passage to sense real airflows with no loss, in contrast to some devices at the previous state of the art that force airflows to follow a curved course with consequent losses that will result in a potentially incorrect reading. Thus, the system according to this invention allows more precise and accurate measurements of inhaled air volumes. Furthermore, the inhalation monitoring device addressed by this invention, and its system, is highly adaptable to various types of inhalers or therapeutic pumps on the market. In preferred embodiments of the inhalation monitoring device addressed by this invention, a flexible part is provided in the area where it slots into the inhaler or therapeutic pump. Thus, the patient can connect a single inhalation monitoring device as addressed by this invention to several different inhalers or therapeutic pump models.

Also the computer-implemented method according to this invention has distinct advantages. For example, the proposed processing of data collected by sensors on the inhalation monitoring device is intended to integrate an assortment of patient data for identifying important patterns indicating potentially more severe conditions of the disease. In particular, the method according to this invention considers personal data such as the patient's gender, age, and weight, as well as the presence or absence of allergens in the geographical area where the patient is located, wherein case it can alert the patient to a possible or probable flare-up of their respiratory disease.

It is also advantageous for the patient to calibrate their inhalation and exhalation without inhaling the drug, whereby data processing and conclusions on inhalation quality are even more accurate, with very low probabilities of the method and the system according to this invention issuing incorrect results.

Advantageously, in certain embodiments of this invention, the inhalation monitoring device according to this invention may be fitted with vibration or motion sensors, such as an accelerometer that detect whether the inhaler or therapeutic pump with the attached inhalation monitoring device (10) is in the correct inhalation position. These vibration and motion sensors collect data that will be processed by the second computing unit when preparing statistics related to the patient.

### BRIEF DESCRIPTION OF THE FIGURES

In order to ensure a proper understanding of the principles underpinning the embodiments of this invention, reference will be made to the embodiments shown in the illustrations and the language used to describe them. It must nevertheless be understood that there is no intention of limiting the scope of this invention to the content presented in the Figures. Any subsequent alterations or modifications to the inventive features illustrated herein, as well as any further applications of the illustrated principles and embodiments of the invention that would normally occur to an expert versed in the matter and in possession of this Specification, are deemed to fall within the scope of the invention claimed herein.
Figure 1 - schematic illustration of an embodiment of the steps constituting the method and the system according to this invention;
Figure 2 - illustration of two front perspective views of two therapeutic pumps attached to two embodiments of the inhalation monitoring device according to this invention;
Figure 3 - illustration of a side view of a therapeutic pump and an embodiment of the inhalation monitoring device according to this invention not connected to the therapeutic pump;
Figure 4 - illustration of two embodiments of the inhalation monitoring device connected to a therapeutic pump and an embodiment of the inhalation monitoring device casing according to this invention;
Figure 5 - illustration of another side view of two therapeutic pumps with two embodiments of the connected inhalation monitoring device according to this invention;
Figure 6 - schematic illustration of the main components of an embodiment of the system according to this invention;
Figure 7 - illustration of an example airflow or flowrate measurement graph for an inhalation and exhalation with an embodiment of the inhalation monitoring device according to this invention.

### DESCRIPTION OF EMBODIMENTS

The technological solution presented by this invention relates to a computer-implemented method and system that performs the method. The system comprises three main elements: an inhalation monitoring device to be used by a patient with asthma, or some other respiratory disease that requires inhaled medication, for inhaling a drug through a therapeutic pump that monitors inhalation quality; a second computing unit, typically the patient's smartphone; and a third computing unit, typically a server.

The technological solution presented by this invention allows drug inhalation quality to be monitored from a therapeutic pump, by connecting an inhalation monitoring device that is connected thereto. The inhalation monitoring device comprises sensors configured to measure the airflow inhaled by a patient using the therapeutic pump and may comprise vibration and motion sensors that collect data on the position of the inhaler or therapeutic pump. The inhalation monitoring device also includes means for storing the data collected by the sensors and means of processing said data, wherein the processing results in the activation of an indicator light, such as a Light Emitting Diode (LED) light in different colors, depending on the message that the inhalation monitoring device intends to convey to the patient. For example, if the inhalation was performed well or was effective, the LED light may be green; if the inhalation was not performed well or was not effective, the LED light may be red. The data is processed by a first computing unit or microprocessor inside the inhalation monitoring device casing, based on data collected by the sensors, particularly data collected by the differential pressure sensors, with these measurements used to calculate the metrics that define inhalation quality, such as Peak Inspired Flow (PIF), first second of Inspired Vital Flow (FIV1), Inspired Vital Capacity (IVC), and Airway Resistance (AWR). Calculation of these metrics includes comparing data collected by the sensors with patient's baseline data and/or baseline data obtained from an appropriate database. The inhalation monitoring device also comprises a communication unit configured to send the inhalation data and calculated metrics to a second computing unit, typically the patient's smartphone, or a desktop computer, laptop, or tablet, where a computer application is installed, suitable for processing the data received from the inhalation monitoring device on the last inhalation, with updated statistics related to the patient's treatment, equipped with a user interface, such as a screen. For the data to be sent to the patient's smartphone, the inhalation monitoring device, and the smartphone, or second computing unit, must be within a nearby field, and they must be paired through wired or wireless means, preferably by wireless means such as bluetooth, Wi-Fi or some other appropriate means of communication, such as Zigbee. When the patient performs an inhalation away from their smartphone, tablet, laptop, or the second computing unit, the inhalation monitoring device stores the data and calculated metrics in its memory for sending later to the second computing unit, once communications between them are re-established. Consequently, the patient's treatment or the inhalation does not depend on their smartphone, ensuring that data collected on such inhalations are not lost. The inhalation monitoring device also comprises a power unit, which may be a replaceable power cell or a chargeable or non-rechargeable battery, with the power source rechargeable through USB Type-C, mᵢni USB, micro USB, or USB Type-A ports, among others.

After processing by the second computing unit, e.g. the patient's smartphone, the latter sends all updated data to a third computing unit, e.g., a cloud server or set of servers, including updated statistics displayed on the screen of the second computing unit and data on geographical location and the respective weather conditions, if authorized to do so by the patient, for storage in a patient database.

In preferred embodiments of this invention, the third computing unit uses an algorithm or set of logic instructions to train a machine learning artificial intelligence model, wherein the following input data are used for the said training: patient data entered through a user interface on the second computing unit; patient-related reference data that may be retrieved from an existing database known to an expert versed in the art (1); baseline data collected during a previous patient training step, performed with the aid of a computer application hosted on the second computing unit, such as the patient's smartphone, for performing an inhalation correctly; data from this inhalation and previous inhalations stored in the third computing unit, such as inhalation date and time, allergens at the patient's location, weather conditions, and patient-reported symptoms entered through the user interface on the second computing unit, calculated metrics for this inhalation, and metrics for previous inhalations, whereby these data are associated with different synaptic weights. In preferred embodiments of this invention, when training the above-mentioned artificial intelligence model, data to which more significant synaptic weights are assigned are typically patient symptoms; metrics for the last inhalation and previous inhalations; and also patient data, such as the number of skipped/taken administrations according to the physician's prescription; and the number of inhalations performed correctly/incorrectly. The output data of the model trained by the method according to this invention are as follows: identification of potential asthma flare-up triggering agents; prediction of potential improvement or worsening of the condition being treated, with the latter leading to loss of control over the disease and potentially more severe asthma attacks.

This invention may be used by the patients themselves or the inhalation monitoring device may be administered by a patient caregiver to children and patients who are conscious but with motor or other constraints.

The preferred embodiments of this invention will now be described through references to the attached Figures, which do not limit the invention in any manner whatsoever to the embodiments represented thereby. This invention is defined by the Claims appended hereto.

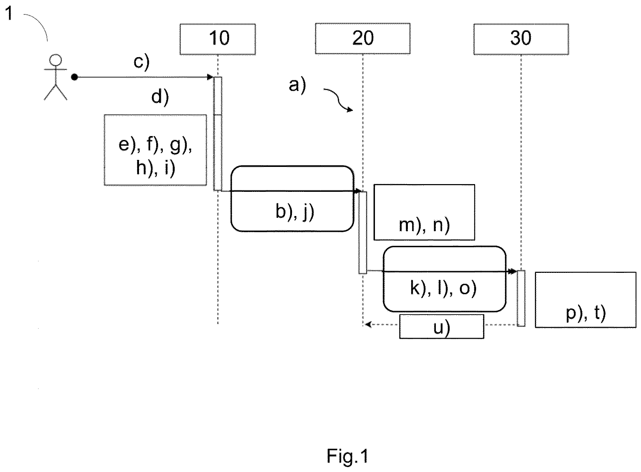

Figure 1 is a schematic illustration of an embodiment of the steps of the method and the system according to this invention. In particular, Figure 1 schematically illustrates the steps of the method according to this invention, also identifying the elements of the system that perform these steps in preferred embodiments of this invention. Figure 1 illustrates a patient (1), an inhalation monitoring device (10) according to this invention, a second computing unit (20) such as laptop, a desktop computer, tablet, or a patient's or caregiver's smartphone or smartwatch, wherein a computer application or a set of logical and sequential instructions has been installed, according to this invention, which perform certain steps of the method according to this invention, and a third computing unit (30), such as a server, a local network computer, a remote cloud computer, a laptop, a desktop, a tablet, a smartphone, or any other computing device accessible through a communications network such as the Internet, wherein another set of logical and sequential instructions is installed that performs certain steps of the method according to this invention.

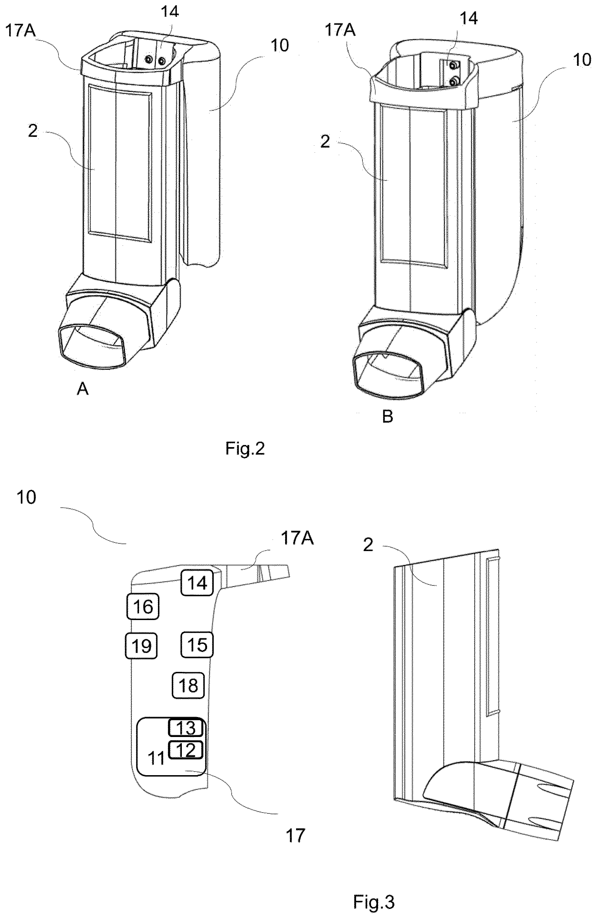

As illustrated in Figure 1, Step a) is performed by the patient (1) through a user interface on the second computing unit (20) and consists of registering the patient by entering patient data through the computer application available in the second computing unit (20). The patient (1) is typically an asthma patient, with chronic obstructive pulmonary disease, cystic fibrosis, or some other respiratory condition that requires inhaled medication. The technological solution presented by this invention may be administered to any patient suffering from any respiratory condition, as mentioned. Such patient data may include, but is not limited to, any of the following: patient's name, caregiver's name, patient's gender/sex, patient's date of birth, patient's height, patient's weight, patient's associated pathologies, patient's or caregiver's email address, patient's or caregiver's telephone number, patient's or caregiver's street address, type of medication taken by the patient, dosages, and administration frequency, patient's geographical location data, one or more symptoms reported by the patient (1), number of doses skipped/taken on time, number of inhalations performed correctly/incorrectly, number of visits to the emergency room, number of times the patient (1) resorted to SOS medication. Schematically illustrated in Figure 1, Step b) shows the pairing between the inhalation monitoring device (10) and the second computing unit (20) through wired or wireless means of communication, preferably wireless, and preferably through bluetooth. During this pairing, the inhalation monitoring device (10) is activated, connected to a therapeutic pump, as described in the following Figures. This activation includes reading reference data for the patient's profile, with these reference data stored in the third computing unit (30) and read by the second computing unit (20). In some embodiments of this invention, the second computing unit (20) may display messages to the patient (1) in the user interface that help the patient perform inhalations correctly and on schedule. For example, these messages may be, and without limitation: reminders to the patient that the time for an inhalation is approaching, and alerts to the patient to keep the SOS medication to hand, due to the detection of potential flare-up trigger situations. These potential situations may be caused by: air quality, atmospheric conditions, and many skipped or performed incorrectly inhalations. The next Step c) is the actual inhalation, performed by the patient (1), with the nozzle of the therapeutic pump inserted into the patient's mouth while exerting pressure on the pressure container with the drug and that is slotted into the therapeutic pump (not shown). In Step d), which is performed at substantially the same time as Step c), inhalation data ddi are collected by one or more differential pressure sensors arrayed on a surface that is tangential to the patient's (1) aspirated airflow, discussed below in Figure 2. Inhalation data are typically as follows: inhalation date and time, airflow, or defined flowrate of one or more differential pressure sensors. Then in Step e), the collected data are stored in a storage unit or memory of the inhalation monitoring device (10, schematically represented in Figure 3, after which metrics mi are calculated for this inhalation, wherein the metrics mi comprise the calculation of Peak Inhalation Flow (PIF), the Forced Inspiratory Volume in one second (FIV1), Inhaled Vital Capacity (IVC) and Airway Resistance (AWR). These metrics are discussed in greater detail below. In Step g) a first computing unit (11) comprised of the inhalation monitoring device (10) processes the inhalation data ddi and the metrics mi calculated for this inhalation, wherein this processing includes a comparison of the data ddi with data from a patient dd₀ baseline and with reference data dd_{ref} retrieved in Step b) above. In particular, this comparison includes comparing the inhalation PIF with the baseline PIF and, depending on the inhalation PIF, the inhalation is rated as compliant or not with the range of values rated as excellent, an efficacious (or non-efficacious) inhalation is defined. The patient's baseline is also compared with the latest inhalation line, as a way of checking the similarity between them. If the inhalation line is similar to the baseline, and efficacious insulation is defined, through similarity metrics, such as, for instance, Euclidean distance or modulation techniques. The remaining IVC, AWR and FIV1 metrics are monitoring metrics, with the pulmonary function assessment and its comparison run against the values registered for previous inhalations, in order to evaluate patient compliance with treatment over time. Next comes Step h) and, depending on the results of the previous step, a light source in the monitoring device casing lights up in one color, such as green, if the result is: *Inhalation Performed Correctly,* with a different color light, such as red, appearing if the result is: *Inhalation Not Performed Correctly.* This indicates that the patient must repeat the inhalation.

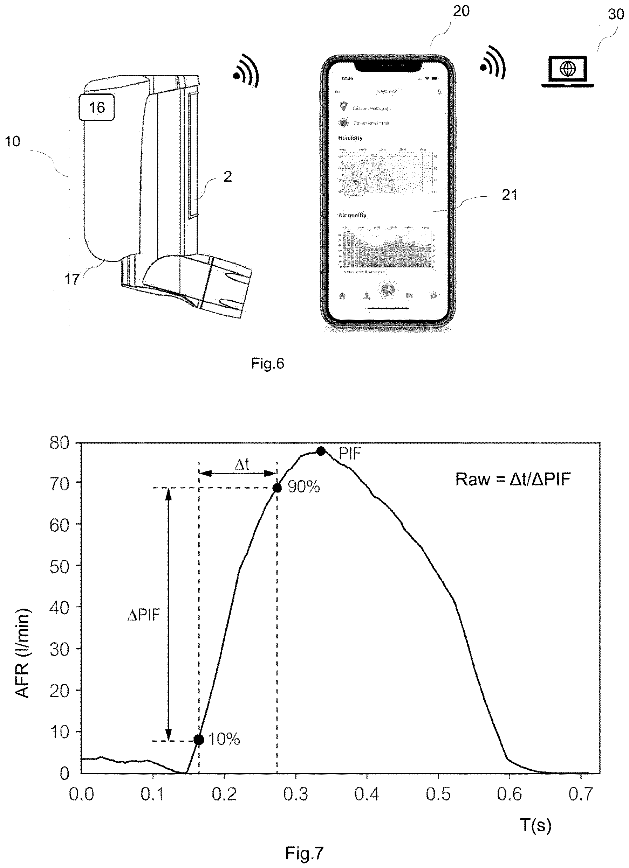

In Step i) shown in Figure 1, the calculated metrics mi are stored by the first computing unit of the inhalation monitoring device (10) in its storage unit or memory. When communication is established with the second computing unit, the communication unit in the inhalation monitoring device (10) sends, in Step k), the data ddi and metrics mi by wired or wireless means, such as through a LAN or WAN, Wi-Fi or other suitable connection, to the third computing unit (30) for further storage in a database and also for further processing. In Step l), data are retrieved from a third computing unit (30) database of at least one of the following types: previous inhalation data ddⱼ, the patient's baseline data dd₀, patient-related reference data dd_{ref}, and metrics mⱼ calculated from previous inhalations by the same patient (1). Next comes Step m)for processing data ddⱼ and the metrics mⱼ wherein this processing is based on a series of comparisons that includes comparing each of the metrics obtained through this inhalation data with identical metrics obtained through data from previous inhalations by the same patient. A comparison is also made between inhalation data ddi obtained by the sensors with patient's baseline data dd₀ and/or reference data dd_{ref}, in order to definition of whether the patient's pulmonary function has evolved positively or negatively, or if there was progress in the patient's pulmonary function. In certain embodiments of the method addressed by this invention, there may be a calibration step prior to Step c) with inhalation, wherein a calibration Step consists of the patient (1) performing an inhalation /exhalation cycle with the therapeutic pump nozzle placed in the patient's (1) mouth and with the inhalation monitoring device (10) connected to the therapeutic pump, with no drug inhalation in this situation, and only patient's baseline data dd₀ collected for use in the calculations that define the inhalation quality, wherein the baseline data dd₀ may be one or more of the following: inhalation/exhalation date and time, inhalation airflow or flowrate, exhalation airflow or flowrate, and symptoms sᵢ reported by the patient. When the patient performs this calibration step, the definition of the correction of subsequent inhalations will be more accurate and less prone to error. In the next Step n), a message is emitted by the second computing unit (20) in the user interface of the second computing unit (20), such as on a smartphone screen, with information about the definition of the progression of the patient's pulmonary function, as discussed below for relation to Figure 6. In Step o), the second computing unit (20) then sends the Step m) processing results to the third computing unit (30), after which the third computing unit (30), such as a server or a set of servers, stores them in a patient (1) related database, in Step p). At this stage, in Step q), the third computing unit (30) calculates statistics related to the progression of the patient's pulmonary function, which may also include the quality of inhalations performed by the patient (1), and may establish a correlation between them. It may also consider other data such as: patient data, inhalation data ddⱼ, patient's baseline data dd₀, position data ddₚ, patient-related reference data dd_{ref}, metrics data mⱼ, geolocation data, weather condition data, air quality data, and patient (1) symptoms data sᵢ. Each time the patient (1) performs an inhalation, Steps (c) to (q) are repeated. Calibration Step c1) may also be performed more than once, as explained above, but it is neither necessary nor essential for this Step to be performed.

In certain embodiments, Step d1) may also be performed to collect position data ddₚ by one or more movement and vibration sensors, such as accelerometers. Data collected by one or more movement sensors in this Step are sent to the second computing unit (20) that defines whether the inhaler or therapeutic pump is in the correct position during inhalation. Should it detect that it was not correctly positioned, a message is emitted in Step n1), which may be displayed on the patient's (1) smartphone screen, with information about the incorrect positioning of the inhalation monitoring device (10) and, in certain embodiments, displaying on the same screen or user interface, the correct position to be used by the patient (1) during inhalation.

In certain embodiments, the user interface of the second computing unit (20) may display a set of tutorial instructions, in a step prior to the inhalation step, which are intended to train the patient (1) in how to improve their inhalation technique. This may include, but is not limited to: instructions on how to place the nozzle of the therapeutic pump in the patient's (1) mouth; instructions on how to inhale and exhale without inhaling the drug in the therapeutic pump; instructions on how to interpret the light source color in the inhalation monitoring device casing (10) after inhalation/exhalation, e.g., green for correct inhalation/exhalation and red for incorrect inhalation/exhalation or inhalation; instructions on how to inhale the drug, and when to do so.

In certain embodiments of this invention, the third computing unit (30), as shown in Figure 1, performs a Step t) of training an artificial intelligence model through machine learning. In this training Step t), the input data are as follows: patient data; patient (1) related reference data dd_{ref}; baseline data dd₀ collected during the calibration step; latest inhalation data ddi; and previous inhalation data ddⱼ, all stored in the third computing unit (30), inhalation date and time; position data ddₚ; allergens at the patient's (1) location, weather conditions, patient's (1) symptoms sᵢ, metrics mᵢ for the latest inhalation; and metrics mⱼ for previous inhalations. The neural networks used FOR training in Step T) work with more significant synaptic weights in some of the data, such as patient (1) symptoms si, metrics mi, metrics mⱼ, and the following patient data: number of administrations skipped/taken on time, and the number of inhalations performed correctly/incorrectly. The output data of the trained model are preferably the following: identification of airborne agents that could potentially trigger flare-ups, such as pollen, dust, humidity, and others, and also predictions of the future progression of the therapeutic condition or possible imminent flare-ups of the respiratory disease symptoms. Finally, in Step u), the results obtained in the artificial intelligence model's training Step t) are sent by the server or the third computing unit (30) to the second computing unit (20), together with the updated statistics

Figure 2 depicts two front views in perspective of two therapeutic pumps (2) with two embodiments of the inhalation monitoring device (10) attached thereto. As shown in Figure 2, the inhalation monitoring device (10) has a connecting element (17A) in a higher area that is configured to fit different therapeutic pump (2) models, whereby it may be easily adapted to any therapeutic pump model. As also shown in Figure 2, on one side of the inhalation monitoring device (10) facing the inside of the therapeutic pump (2) there are two side-by-side apertures (Drawing A in Figure 2) or two vertically arrayed apertures (Drawing B in Figure 2) behind which a differential pressure sensor (14) is installed. This differential pressure sensor (14) is used to measure the airflow when the patient (1) inhales, being adjacent to the surface where the apertures of the differential pressure sensor are located (14). In certain embodiments, more differential pressure sensors (14) may be supplied and, as shown in Figure 2, the sensor apertures (14) may be arrayed in different configurations, such as horizontally or vertically. For the purposes of clarity, the pressure container with the drug is not shown in Figure 2.

Figure 3 shows a side view of the therapeutic pump (2) and the inhalation monitoring device (10), wherein the inner components are represented schematically in certain embodiments. The inhalation monitoring device (10) has a casing (17) designed in manner that adapts to different therapeutic pump (2) models, slotting into it through its element (17A), as already discussed, and positioned optimally whereby the airflow that is breathed in during a calibration performed through Step c1) of the method according to this invention, or the airflow that is breathed in when inhaling the drug, is measured as accurately as possible by the differential pressure sensor or sensors (14). Figure 3 also shows the area where these differential pressure sensors are positioned (14). In certain embodiments, the inhalation monitoring device (10) may additionally comprise one or more vibration or motion sensors (15), such as an accelerometer or a gyroscope or other suitable motion sensor, whereby this vibration or motion sensor (15) is configured to detect the movement of the inhalation monitoring device (10) when the patient (1) grasps it to perform an inhalation, and can help activate a power supply unit (18), such as a power cell or a battery built into the inhalation monitoring device (10), but essentially the vibration and motion sensor (15) detects the position of the inhalation monitoring device (10) during inhalation and collects the position data ddₚ, which will be stored in a storage unit (12), such as a Read Only Memory (ROM), a Random Access Memory (RAM), or some other suitable type of memory, from the inhalation monitoring device (10), as already discussed above in Figure 1. Figure 3 also shows a light source (16) that may comprise one or more Light Emitting Diodes (LEDs) which may emitted light in at least two different colors, depending on the result obtained in metric processing Step g), whereby, for example, if the result corresponds to a correct inhalation, the LED emits a green light, but if the result indicates an incorrect inhalation, the LED emits a red light. As shown in Figure 3, on a rear side of the inhalation monitoring device (10) is a capacitive button (19). This capacitive button (19) activates the inhalation monitoring device (10), meaning that it activates the power supply unit (18), whereby the inhalation monitoring device (10) can start operating when the patient (1) picks it up to perform an inhalation or calibration, as already discussed. The above-mentioned power supply unit (18) of the inhalation monitoring device (10) may be a power cell or a rechargeable or non-rechargeable battery. The inhalation monitoring device (10) comprises a first computing unit (11), such as a microprocessor that converts the analog signals collected from one or more differential pressure sensors (14) and one or more vibration and motion sensors (15) into digital signals, and the first computing unit (11), as already discussed in Figure 1, calculates the metrics mi based on the ddi values measured by the differential pressure sensors (14), storing them in the storage unit (12), or memory, also depicted in Figure 3. The inhalation monitoring device (10) also comprises a communication unit (13) that communicates by wired or wireless means, preferably wirelessly, such as bluetooth, wi-fi, or zigbee, preferably bluetooth, with the second computing unit (20) of the invention system reporting the data ddi and data dd₀ obtained from a calibration performed by the patient (1) and metrics mi. The communication unit (13) also pairs with the second computing unit (b), as discussed above.

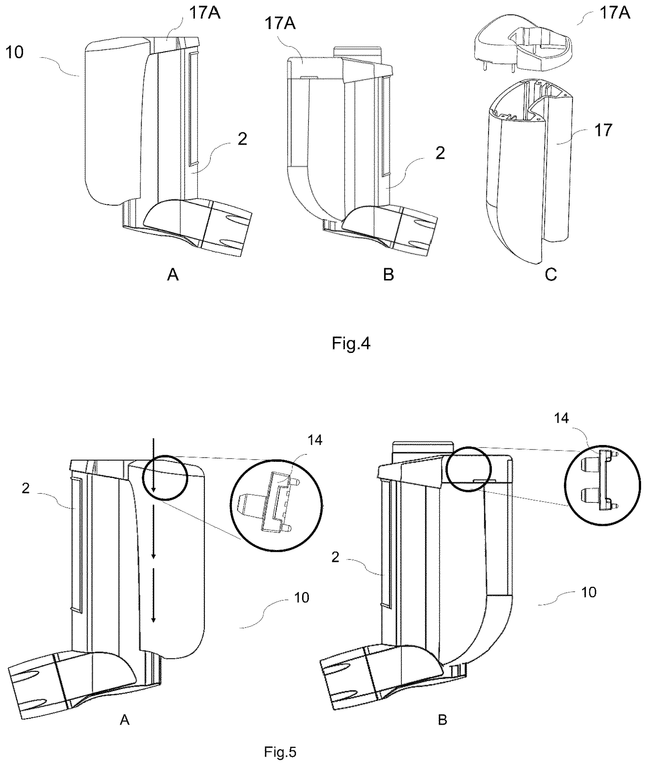

Figure 4 shows a side view of two embodiments of the inhalation monitoring device (10) connected to a therapeutic pump (2) (drawings A and B), through the connecting element (17A). Figure 4 further shows an embodiment (Drawing C) of the casing (17) wherein the connecting element (17A) is detachable, to allow an easier and more efficient slotting of the inhalation monitoring device (10) into the therapeutic pump (2). Drawing A in Figure 4 shows the slotting of the inhalation monitoring device (10) onto the therapeutic pump before inserting the pressure vessel with the drug. Drawing B of Figure 4 shows the pressure vessel with the drug inserted into the therapeutic pump (2). These drawings thus illustrate the diversity of therapeutic pump models (2) to which the inhalation monitoring device (10) may be attached.

Figure 5 illustrates side views of two embodiments of the inhalation monitoring device (10) connected to a therapeutic pump (2), where a detail of a side view of a differential pressure sensor (14) is shown. In Drawing A of Figure 5, the differential pressure sensor (14) has the pressure outlet points arrayed horizontally and in Drawing B of Figure 5, the differential pressure sensor (14) has the pressure outlet points arrayed vertically. In both types of embodiments illustrated in Figure 5, the differential pressure sensor (14) is optimally positioned to measure the incoming airflow adjacent to the surface behind which it is installed, when the patient (1) breathes in or performs an inhalation. Drawing A of Figure 5 illustrates the said airflow through the vertical downward arrows.

Figure 6 shows a side view of an embodiment of the inhalation monitoring device (10) connected to a therapeutic pump (2) with an embodiment of the second computing unit (20), in this case, the patient's (1) smartphone, and an embodiment of a third computing unit (30), in this example, a remote server. As already discussed in Figure 1, the inhalation monitoring device (10) communicates with the second computing unit (20), which in turn communicates with the third computing unit (30) by wired or wireless means, as already discussed. Figure 6 shows a user interface (21), in this case a patient's (1) smartphone screen, displaying data on air quality, pollen, and humidity in the geographic area where the patient (1) is located. This user interface (21) may also be used to enter data related to symptoms (not shown) experienced by the patient (1) on a screen that displays aspects related to the patient's symptoms.

Figure 7 shows a graph with an example of inhalation and exhalation values measured by the differential pressure sensor (14) in accordance with this present invention and as shown in Figure 3, wherein the graph presents the Forced Inspiratory Volume (FIV1). The abscissa axis represents the time in seconds, and the ordinate axis represents the airflow or flowrate measured in liters per minute (L/min). The curve represents the inhalation performed by the patient (1). The upper point identified as the Peak Inspired Flow (PIF) is one the metrics mi calculated by the first computing unit (11) of the inhalation monitoring device (10) In The Coordinates with the invention. The graph in Figure 7 also shows the AWR = Δt/ΔPIF metric (AWR = Airway Resistance), representing the ratio between the period from time when the patient begins to breathe in, from 10% of the PIF up to 90% of the PIF, providing an airway resistance for the patient (1). Another metric calculated by the first computing unit (11) is the Inspired Vital Capacity (IVC), which represents the area under the curve in the graph shown in Figure 7. The PIF is the most important metric for classifying inhalation quality. Depending on whether or not the PIF value of the inhalation falls within the range of values rated as excellent, bearing in mᵢnd the therapeutic pump used and the patient data (such as: age, gender), the inhalation is rated as efficacious or not efficacious respectively. The IVC metric responds to the forced air volume inhaled when breathing in normally, and may be extracted from the inhalation profile, being directly proportional to the PIF. The comparison between the inhalation IVC and the values for previous inhalations allows an assessment of the progression of the patient's pulmonary capacity, as good compliance with treatment is reflected in a gradual increase (or maintenance) of the IVC over time, and consequently an increase (or maintenance) of the PIF. The AWR metric corresponds to the airflow resistance of the respiratory tract during inhalation and exhalation. This is also a metric that allows an assessment of pulmonary capacity progression, as good compliance with treatment is reflected in a gradual reduction (or maintenance) of the AWR.

In a first aspect, the invention relates to a computer-implemented method for monitoring the quality of inhalations performed by a patient (1) using a therapeutic pump (2) containing a drug, wherein the therapeutic pump (2) is connected to an inhalation monitoring device (10), with the said method comprising the following steps:
a) patient data recorded in a second computing unit (20) by entering patient (1) data through a user interface (21) in the second computing unit (20);
b) activation of an inhalation monitoring device (10) properly connected to a therapeutic pump (2), wherein the activation comprises the following substeps:
   b1) detection by a capacitive button (19) inside the inhalation monitoring device (10) of the start of a patient's use of the inhalation monitoring device (10);
   b2) start-up of powering the inhalation monitoring device (10) by the power supply unit (18);
   b3) pairing between the said inhalation monitoring device (10) through a communication unit (13) and the second computing unit (20) by wired or wireless communication;
   b4) the second computing unit (20) reads reference data dd_{ref} corresponding to the patient (1) from a database hosted in a third computing unit (30);
c) with the therapeutic pump (2) nozzle inserted into the patient's (1) mouth, the patient (1) engages in the inhalation of a drug by pressing the therapeutic pump (2);
d) inhalation data ddi collection by the inhalation monitoring device (10) through a set of sensors (14);
e) storage of the inhalation data ddi collected in Step d) by the first computing unit (11) in a storage unit (12) of the inhalation monitoring device (10);
f) calculation, by the first computing unit (11), of metrics based on the inhalation data ddi collected in Step d), where the calculation comprises the definition of at least one of the following metrics mi:
   - Peak Inspired Flow (PIF)
   - Forced Inspiratory Volume in one second (FIV1)
   - Inspired Vital Capacity (IVC)
   - Airway Resistance (AWR);
g) processing of data ddi and metrics mi by a first computing unit (11), wherein the processing includes the following substep:
   g1) comparison of at least one of the obtained metrics mi with a patient's baseline dd₀ and/or reference data dd_{ref};
   wherein the comparison of Substep G1) allows:
      - definition of whether the inhalation was performed correctly; and
      - definition of whether the patient needs to repeat the inhalation;
h) emission of a visual alert by a light source (16) in the casing (17) of the inhalation monitoring device (10), whereby the light has a first color when the inhalation is defined as completed correctly, and the light has a second color when the inhalation is defined as not completed correctly;
i) storage of the metrics mi calculated in Step f), by the above-mentioned first computing unit (11), in the storage unit (12) of the inhalation monitoring device (10);
j) sending the inhalation data ddi collected in Step d) and the metrics mi calculated in Step f) by cable or wireless means from a communication unit (13) of the inhalation monitoring device (10) to the second computing unit (20);
k) sending the inhalation data ddi and metrics mi from the second computing unit (20) to a third computing unit (30) for database storage in a third computing unit memory (30);
l) retrieval from a database in a third computing unit (30) of at least one type of data from the group comprised of: inhalation data ddⱼ, patient's baseline data dd₀, patient-related reference data dd_{ref}, and metrics mⱼ;
   wherein the inhalation data ddⱼ and metrics mⱼ refer to inhalation data and metric data calculated for previous inhalations by the same patient (1);
m) processing the inhalation data ddⱼ and metrics mⱼ obtained through the retrieval in the previous step by the second computing unit (20), wherein the processing includes the following substeps:
   m1) comparison of each metric datum mi of this inhalation with metrics mⱼ from previous inhalations;
   m2) comparison of data from this inhalation data ddi with the patient's baseline data dd₀ and/or with reference data dd_{ref};
   wherein comparisons of substeps m1) and m2) allow:
      - definition of whether the patient's pulmonary function has evolved positively or negatively, or if there has been no progression in the patient's pulmonary function;
n) issuing a message on a user interface (21) of the second computing unit (20) with information on the definition of the progression of the patient's (1) pulmonary function obtained in the previous step;
o) sending the results obtained in Step m) from the second computing unit (20) to the third computing unit (30), for storage in the above-mentioned database of the third computing unit (31);
p) storage by the third computing unit (30) of the data sent in Step o) in a database hosted in the third computing unit (30);
q) calculation, by the third computing unit (30), of statistics associated with the progression of the patient's pulmonary function, the quality of inhalations performed by the patient (1), and the correlation between them, with at least one of the following used in the statistics: patient data, inhalation data ddⱼ, patient's baseline data dd₀, position data ddₚ, patient-related reference data dd_{ref}, metrics data mⱼ, geolocation data, weather conditions data, air quality data, and patient (1) symptoms data si;
wherein steps c) to q) are repeated with each inhalation by the patient (1) using the therapeutic pump (2).

In an embodiment of the first aspect of this invention, the computer-implemented method described above includes the following additional steps:
c1) calibration of the inhalation monitoring device (10) with the inhalation monitoring device (10) connected to the therapeutic pump (2), wherein such calibration consists of the patient (1) completing an inhalation and exhalation cycle with the nozzle of the therapeutic pump (2) placed in the patient's (1) mouth and without inhaling the drug from the therapeutic pump (2), whereby the patient's baseline data dd₀ (1) are obtained during this calibration (130);
wherein
Step c1) is performed before Step d);
wherein Storage Step e) includes storing the patient's (1) baseline data dd₀ in the storage unit (12) of the inhalation monitoring device (10);
Sending Step j) includes sending baseline data from the patient's (1) baseline data dd₀ to the second computing unit (20);
Sending Step k) includes sending the patient's (1) baseline data dd₀ to the third computing unit (30).

In yet another embodiment of the first aspect of this invention, the computer-implemented method described above includes the following additional steps:
d1) collection of position data ddₚ by the inhalation monitoring device (10) through a set of motion sensors (15); and
(n1) issuing a message on a user interface (21) of the second computing unit (20) with information on the incorrect positioning of the inhalation monitoring device (10);
wherein Step d1) is performed at substantially the same time as Step d),
wherein Storage Step e) includes storing the inhalation position data ddₚ in the storage unit (12) of the inhalation monitoring device (10);
wherein Sending Step j) includes sending the position data ddₚ collected in Step d1) to the second computing unit (20);
wherein Sending Step k) includes sending the position data ddₚ collected in Step d1) to the third computing unit (30).

In another embodiment of the first aspect of this invention, the computer-implemented method described above includes the following additional step:
a0) Display on a user interface (21) of the second computing unit (20) of a tutorial comprising at least one instruction from the group comprised of:
   - instruction on how to place the nozzle of the therapeutic pump (2) in the patient's (1) mouth;
   - instruction on how to inhale and exhale without inhaling the drug in the therapeutic pump (2);
   - instruction on how to interpret the color of the light source in the casing (17) of the inhalation monitoring device (10) after inhalation/exhalation;
   - instruction on how and when to inhale the drug contained in the therapeutic pump (2);
   - instruction on how to interpret the color of the light source (16) in the casing (17) of the inhalation monitoring device (10) after inhalation,
wherein step a0) is performed after Activation Step b).

In another embodiment of the first aspect of this invention, the computer-implemented method described above includes the following additional steps:
s) display on a user interface (21) on the second computing unit (20) of a message requesting the entry of symptoms experienced by the patient (1); and
s1) entry (265) by the patient (1) of one or more symptoms experienced by the patient (1) through the user interface (21) on the second computing unit (20);
wherein steps s) and s1) are performed before or after any of the steps encompassed by the method.

In another embodiment of the first aspect of this invention, the computer-implemented method described above includes the following additional steps:
t) Training an artificial intelligence logical instruction model, wherein Training Step t) is performed by the third computing unit (30) and uses the following input data:
   - Patient data entered in Step a);
   - reference data dd_{ref} related to the patient (1);
   - baseline data dd₀ collected in step c1);
   - Data ddi from this inhalation and previous inhalations ddⱼ collected in Step d) and stored in the third computing unit (30), respectively;
   - date and time of inhalation;
   - position data ddₚ;
   - allergens in the patient's (1) location;
   - weather conditions;
   - the patient's (1) symptoms si;
   - metrics mi referring to this inhalation and metrics mⱼ referring to previous inhalations;
wherein the following data have the most significant synaptic weights: patient (1) symptoms sᵢ, metrics mi, metrics mⱼ, and the following patient data: number of administrations skipped/taken on time, number of inhalations performed correctly/incorrectly;
wherein the output data of the trained model is as follows:
   - identification of potential flare-up triggering agents;
   - prediction of potential worsening of the therapeutic condition that may cause an uncontrolled disease and potential flare-ups; and
u) sending of the results obtained in the previous step from the third computing unit (30) to the second computing unit (20), together with the updated statistics, calculated for the patient (1)in Step q).

In another embodiment of the first aspect of this invention, the patient data entered in Step a) of the computer-implemented method described above comprise at least one of the group comprised of: patient's name, caregiver's name, patient's gender/sex, patient's date of birth, patient's height, patient's weight, patient's associated pathologies, patient's or caregiver's e-mail address, patient's or caregiver's contact telephone number, patient's or caregiver's street address, type of medication taken by the patient, its dosage and administration frequency, details of the patient's geographical location, one or more symptoms reported by the patient (1), number of doses skipped/taken at the right time, number of inhalations performed correctly/incorrectly, number of emergency room visits, number of times the patient (1) resorted to SOS medication.

In yet another embodiment of the first aspect of this invention, the pairing between the inhalation monitoring device (10) and the second computing unit (20), by wired or wireless communication, performed in Step b) of the computer-implemented method described above includes the patient (1) entering authorization to identify the geographical location of the second computing unit (20) and collect location data during either of the following steps: c) and c1), wherein such location data comprise at least one of the group comprised of: geographical location, weather conditions, and outdoor airborne allergen concentrations.

In yet another embodiment of the first aspect of this invention, when Step c1) is performed, the baseline data dd₀ obtained during the above-mentioned Step c1) comprise at least one of the group comprised of: inhalation/exhalation date and time, inhalation airflow or flowrate, exhalation airflow or flowrate, symptoms reported by the patient (1).

In yet another embodiment of the first aspect of this invention, the inhalation data collected in Step f) of the computer-implemented method comprise at least one of the group comprised of: inhalation date and time, airflow, or flowrate.

In another embodiment of the first aspect of this invention, the computer-implemented method described above includes the following additional step before Step c):
c0) display of at least one message from the group comprised of:
- message reminding the patient of an approaching inhalation time; and
- alert warning the patient to carry the SOS medication with them due to the detection of potential flare-up trigger situations, with such potential situations being at least one of the group comprised of: air quality, atmospheric conditions, many inhalations skipped or performed incorrectly.

In yet another embodiment of the first aspect of this invention, the patient is a person living with asthma, chronic obstructive pulmonary disease, cystic fibrosis, or with another respiratory condition that requires inhaled medication.

In a second aspect, the invention relates to a system configured to implement the method as defined above in relation to the first aspect of the invention, whereby such system comprises:
- An inhalation monitoring device (10) configured for connection to a therapeutic pump (2) and used by a patient during inhalation/exhalation or for inhalation with the said therapeutic pump (2), wherein the inhalation monitoring device (10) comprises:
   - a casing (17) configured for connection to the said therapeutic pump (2);
   - a power supply unit (18) configured to power the inhalation monitoring device (10);
   - a first computing unit (11) of the inhalation monitoring device (10);
   - a data storage unit (12) configured to store data collected and/or processed by the inhalation monitoring device (10);
   - a set of sensors (14) comprising one or more differential pressure sensors, configured to measure the differential airflow;
   - at least one capacitive button (19) configured to activate the inhalation monitoring device (10);
   - a communication unit (13) set up to communicate through wired or wireless means with a second computing unit (20);
   - a connecting element (17A) of the inhalation monitoring device (10), wherein the connecting element is arrayed in the casing (17);
   - at least one light source (16) arrayed in the casing (17) and configured to emit light in at least two distinct colors, a first color and a second color;
   - second computing unit (20), wherein the second computing unit (20) comprises:
   - a user interface (21) configured to display information to the user, such as being configured to display instructions for an inhalation tutorial and receive user input, and for user data entry, wherein the user may be either the patient (1) or a patient (1) caregiver

wherein the second computing unit (20) is selected from the group comprised of: laptop, desktop computer, tablet, smartphone, smartwatch; and
wherein the second computing unit (20) is set up to communicate with the communication unit (13) of the inhalation monitoring device (10) and with a third computing unit (30), by wired or wireless means of communication;
   - third computing unit (30), wherein the third computing unit (30) is a device selected from the group comprised of: server, local network computer, remote cloud computer, laptop, desktop computer, tablet, smartphone or any other computing device accessible through a communications network such as the Internet, wherein the third computing unit (30) is set up to communicate with the second computing unit (20) by wired or wireless means.

In an embodiment of the second aspect of the invention, the inhalation monitoring device (10) additionally comprises:
- a set of motion sensors (15) comprising one or more vibration and motion sensors configured to measure the position of the inhalation monitoring device (10) when connected to the therapeutic pump (2),
wherein the data captured by the sensor array (15) are sent in Step j) from the communication unit (13) of the inhalation monitoring device (10) to the second computing unit (20) and are then sent in Step k) from the second computing unit (20) to the third computing unit (30).

In a third aspect, the invention relates to a computer program product comprising a set of logic instructions whereby, when the program is run on a computer, such as the first computing unit (11) of the system as defined in the second aspect of the invention, the computer performs the steps of the method as defined in the first aspect of this invention.

In a fourth aspect, the invention relates to a computer program product comprising a set of logic instructions whereby, when the program is run on a computer, for example by the second computing unit (20) of the system as defined in the second aspect of the invention, the computer performs the steps of the method as defined in the first aspect of this invention.

In a fifth aspect, the invention relates to a computer program product comprising a set of logic instructions whereby, when the program is run on a computer, for example by the third computing unit (30) of the system as defined in the second aspect of the invention, the computer performs the steps of the method as defined in the first aspect of this invention.

In a sixth aspect, the invention relates to a computer-readable storage medium comprising the installation of a computer program product as defined in the third, fourth and fifth aspects of the invention.

### DEFINITIONS

As used throughout this patent application, the term "or" is used in an inclusive rather than an exclusive sense, unless the exclusive meaning is clearly defined in a specific situation. In this context, a sentence such as "X uses A or B" shall be interpreted as including all relevant inclusive combinations, such as "X uses A", "X uses B" and "X uses A and B".

As used throughout this patent application, the indefinite articles "a/an" or "one" shall generally be interpreted as "a/or more" and "one or more", unless the meaning of a singular form of an embodiment is clearly defined in a specific situation.

As presented in this Specification, terms related to examples shall be interpreted for the purpose of illustrating an example of something, rather than indicating a preference.

The subject matter described above is provided as an illustration of this invention, and shall not be construed as imposing limitations thereon. The terminology used for the purpose of describing specific embodiments in accordance with this invention shall not be construed as limiting the invention. As used in the Specification, definite and indefinite articles, in their singular form, shall be interpreted as also including their plural forms, unless the context of the Specification explicitly indicates otherwise. It shall be understood that, when used in this Specification, the terms "understand" and "include" define the presence of the characteristics, elements, components, steps, and related operations, but do not exclude the possibility that other characteristics, elements, components, steps, and operations may also be encompassed thereby.

As long as they do not modify the essential characteristics of the following Claims, all modifications, shall be deemed to fall within the scope of the protection sought for this invention.

### LIST OF REFERENCE INDICATIONS

1 - Patient
2 - Therapeutic pump
10 - Inhalation Monitoring Device
11 - First Computing unit
12 - Storage Unit
13 - Communication Unit
14 - Differential Pressure Sensor
15 - Motion Sensor
16 - LED Light Source
17 - Casing
17A - Connecting Element
18 - Power Supply Unit
19 - Capacitive Button
20 - Second Computing Unit
21 - User Interface
30 - Third Computing Unit
a) - Step a) of the method used in the invention
b) - Step b) of the method used in the invention
c) - Step c) of the method used in the invention
d) - Step d) of the method used in the invention
e) - Step e) of the method used in the invention
f) - Step f) of the method used in the invention
g) - Step g) of the method used in the invention
h) - Step h) of the method used in the invention
i) - Step i) of the method used in the invention
j) - Step j) of the method used in the invention
k) - Step k) of the method used in the invention
l) - Step l) of the method used in the invention
m) - Step m) of the method used in the invention
n) - Step n) of the method used in the invention
o) - Step o) of the method used in the invention
p) - Step p) of the method used in the invention
q) - Step Q) of the method used in the invention
f) - Step t) of the method used in the invention
u) - Step u) of the method used in the invention
PIF - Peak Inspiratory Flow
AWR - Airway Resistance
AFR - Airflow Rate
T (s) - time in seconds

### LIST OF CITATIONS

The list of citations is presented below:

### PATENT LITERATURE

US Patent Application N° 2016/0144141 A1, filed by Cognita Labs, LLC, Houston, TX, USA, published on May 26, 2016.

## Claims

1. A computer-implemented method for monitoring the quality of inhalations performed by a patient (1) using a therapeutic pump (2) containing a drug, wherein the therapeutic pump (2) is connected to an inhalation monitoring device (10), whereby the said method is **characterized in that** it comprises the following steps:
a) patient data recorded in a second computing unit (20) by entering patient (1) data through a user interface (21) in the second computing unit (20);
b) activation of an inhalation monitoring device (10) properly connected to a therapeutic pump (2), wherein the activation comprises the following substeps:
b1) detection by a capacitive button (19) inside the inhalation monitoring device (10) of the start of a patient's use of the inhalation monitoring device (10);
b2) start-up of powering the inhalation monitoring device (10) by the power supply unit (18);
b3) pairing between the said inhalation monitoring device (10) through a communication unit (13) and the second computing unit (20) by wired or wireless communication;
b4) the second computing unit (20) reads reference data dd_{ref} corresponding to the patient (1) from a database hosted in a third computing unit (30);
c) with the therapeutic pump (2) nozzle inserted into the patient's (1) mouth, the patient (1) engages in the inhalation of a drug by pressing the therapeutic pump (2);
d) inhalation data ddi collection by the inhalation monitoring device (10) through a set of sensors (14);
e) storage of the inhalation data ddi collected in Step d) by the first computing unit (11) in a storage unit (12) of the inhalation monitoring device (10);
f) Calculation, by the first computing unit (11), of metrics based on the inhalation data ddi collected in Step d), where the calculation comprises the definition of at least one of the following metrics mi:
- Peak Inspired Flow (PIF)
- Forced Inspiratory Volume (FIV1)
- Inspired Vital Capacity (IVC)
- Airway Resistance (AWR);
g) processing of data ddi and metrics mi by a first computing unit (11), wherein the processing includes the following substep:
g1) comparison of at least one of the obtained metrics mi with a patient's baseline dd₀ and/or reference data dd_{ref};
wherein the comparison of Substep g1) allows:
- definition of whether the inhalation was performed correctly; and
- definition of whether the patient needs to repeat the inhalation;
h) emission of a visual alert by a light source (16) in the casing (17) of the inhalation monitoring device (10), whereby the light has a first color when the inhalation is defined as completed correctly, and the light has a second color when the inhalation is defined as not completed correctly;
i) storage of the metrics mi calculated in Step f), by the above-mentioned first computing unit (11), in the storage unit (12) of the inhalation monitoring device (10);
j) sending the inhalation data ddi collected in Step d) and the metrics mi calculated in Step f) by cable or wireless means from a communication unit (13) of the inhalation monitoring device (10) to the second computing unit (20);
k) sending the inhalation data ddi and metrics mi from the second computing unit (20) to a third computing unit (30) for database storage in a third computing unit memory (30);
l) retrieval from a database in a third computing unit (30) of at least one type of data from the group comprised of: inhalation data ddⱼ, patient's baseline data dd₀, patient-related reference data dd_{ref}, and metrics mⱼ;
wherein the inhalation data ddⱼ and metrics mⱼ refer to inhalation data and metric data calculated for previous inhalations by the same patient (1);
m) processing the inhalation data ddⱼ and metrics mⱼ obtained through the retrieval in the previous step by the second computing unit (20), wherein the processing includes the following substeps:
m1) comparison of each metric datum mᵢ of this inhalation with metrics mⱼ from previous inhalations;
m2) comparison of data from this inhalation data ddi with the patient's baseline data dd₀ and/or with reference data dd_{ref};
wherein comparisons of substeps m1) and m2) allow:
- definition of whether the patient's pulmonary function has evolved positively or negatively, or if there has been no progression in the patient's pulmonary function;
n) issuing a message on a user interface (21) of the second computing unit (20) with information on the definition of the progression of the patient's (1) pulmonary function obtained in the previous step;
o) sending the results obtained in Step m) from the second computing unit (20) to the third computing unit (30), for storage in the above-mentioned database of the third computing unit (31);
p) storage by the third computing unit (30) of the data sent in Step o) in a database hosted in the third computing unit (30);
q) calculation, by the third computing unit (30), of statistics associated with the progression of the patient's pulmonary function, the quality of inhalations performed by the patient (1), and the correlation between them, with at least one of the following used in the statistics: patient data, inhalation data ddⱼ, patient's baseline data dd₀, position data ddₚ, patient-related reference data dd_{ref}, metrics data mⱼ, geolocation data, weather conditions data, air quality data, and patient (1) symptoms data si;
wherein steps c) to q) are repeated with each inhalation by the patient (1) using the therapeutic pump (2).

2. The computer-implemented method according to Claim 1, **characterized in that** it comprises the following steps:
c1) with the inhalation monitoring device (10) connected to the therapeutic pump (2), calibration of the inhalation monitoring device (10), whereby such calibration consists of the patient (1) completing an inhalation and exhalation cycle with the nozzle of the therapeutic pump (2) placed in the patient's (1) mouth and without inhaling the drug from the therapeutic pump (2), whereby the patient's baseline data dd₀ (1) are obtained during this calibration (130);
wherein
Step c1) is performed before Step d);
Storage Step e) includes storing patient's baseline data dd₀ (1) in the storage unit (12) of the inhalation monitoring device (10);
Sending Step j) includes sending patient's baseline data dd₀ (1) to the second computing unit (20);
sending step k includes sending patient's baseline data dd₀ (1) from the to the third computing unit (30).

3. The computer-implemented method according to any one of the preceding Claims, **characterized in that it** also comprises the following steps:
d1) collection of position data ddₚ by the inhalation monitoring device (10) by means of a set of motion sensors (15); and
n1) issuing a message on a user interface (21) of the second computing unit (20) with information on the incorrect positioning of the inhalation monitoring device (10);
wherein Step d1)is substantially performed simultaneously with Step d),
wherein Storage Step e) includes storing the inhalation position data ddₚ in the storage unit (12) of the inhalation monitoring device (10);
wherein Sending Step j) includes sending the position data ddₚ collected in Step d1), to the second computing unit (20);
wherein Sending Step k) includes sending the position data ddₚ collected in Step d1), to the third computing unit (30).

4. The computer-implemented method according to any one of the preceding Claims, **characterized in that it** also comprises the following step:
(a0) Display in a user interface (21) of the second computing unit (20) of a tutorial comprising at least one instruction from the group comprised of:
- instruction on how to place the nozzle of the therapeutic pump (2) in the patient's (1) mouth;
- instruction on how to inhale and exhale without inhaling the drug inside the therapeutic pump (2);
- instruction on how to interpret the color of the light source in the casing (17) of the inhalation monitoring device (10) after inhalation/exhalation;
- instruction on how and when to inhale the drug contained in the therapeutic pump (2);
- instruction on how to interpret the color of the light source (16) in the casing (17) of the inhalation monitoring device (10) after inhalation,
wherein step a0) is performed after Activation Step b).

5. The computer-implemented method according to any one of the preceding Claims, **characterized in that it** also comprises the following steps:
s) display in a user interface (21) of the second computing unit (20) of a message requesting the entry of symptoms experienced by the patient (1); and
s1) entry (265) by the patient (1) of one or more symptoms experienced by the patient (1) through the user interface (21) of the second computing unit (20);
wherein steps s) and s1) are performed before or after any of the steps of the method.

6. The computer-implemented method according to any one of the preceding Claims, **characterized in that it** also comprises the following steps:
u) Training an artificial intelligence logical instruction model, wherein Training Step t) is performed by the third computing unit (30) and uses the following input data:
- Patient data entered in Step a);
- reference data dd_{ref} related to the patient (1);
- baseline data dd₀ collected in step C1);
- Data from this ddi inhalation and previous ddⱼ inhalations collected in Step d) and stored in the third computing unit (30), respectively;
- date and time of inhalation;
- position data ddₚ;
- allergens in the patient's (1) location;
- weather conditions;
- Patient's (1) symptoms si;
- metrics mi referring to this inhalation and metrics mⱼ referring to previous inhalations;
wherein the following data have the most significant synaptic weights: patient symptoms sᵢ(1), metrics mi, metrics mⱼ, and the following patient data: number of administrations skipped/taken on time, number of inhalations performed correctly/incorrectly;
wherein the output data of the trained model is as follows:
- identification of potential flare-up triggering agents;
- prediction of potential worsening of the therapeutic condition that may cause an uncontrolled disease and potential flare-ups; and
u) Sending by the third computing unit (30), to the second computing unit (20) of the results obtained in the previous step and the updated statistics, calculated in Step q) for the patient (1).

7. The computer-implemented method according to any one of the preceding Claims, **characterized in that the** patient data entered in Step a) encompass at least one of the group comprised of: patient's name, caregiver's name, patient's gender/sex, patient's date of birth, patient's height, patient's weight, patient's associated pathologies, patient's or caregiver's e-mail address, patient's or caregiver's contact telephone number, patient's or caregiver's street address, type of medication taken by the patient, its dosage and administration frequency, details of the patient's geographical location, one or more symptoms reported by the patient (1), number of doses skipped/taken at the right time, number of inhalations performed correctly/incorrectly, number of emergency room visits, number of times the patient (1) resorted to SOS medication.

8. The computer-implemented method according to any one of the preceding Claims, **characterized in that the** pairing between the inhalation monitoring device (10) and the second computing unit (20), by wired or wireless communication, performed in Step b) include the patient (1) keying in authorization to identify the geographic location of the second computing unit (20) and the respective collection of location data, during any of Steps (c) and c1), wherein the location data comprise at least one of the group comprised of: geographical location, weather conditions, and outdoor airborne allergen concentrations.

9. The computer-implemented method according to any one of Claims 2 to 8, **characterized in that the** baseline data dd₀ obtained in Step c1) encompass at least one of the group comprised of: inhalation/exhalation date and time, inhalation airflow or flowrate, exhalation airflow or flowrate, symptoms reported by the patient (1).

10. The computer-implemented method according to any one of the preceding Claims, **characterized in that the** inhalation data ddi collected in Step f) encompass at least one of the group comprised of: inhalation date and time, airflow, or flowrate.

11. The computer-implemented method according to any one of the preceding Claims, **characterized in that** it comprises the following additional step, before Step c):
c0) display of at least one message from the group comprised of:
- message reminding the patient of an approaching inhalation time; and
- alert warning the patient to carry the SOS medication with them due to the detection of potential flare-up trigger situations, with such potential situations being at least one of the group comprised of: air quality, atmospheric conditions, many inhalations skipped or performed incorrectly.

12. The computer-implemented method according to any one of the preceding Claims, **characterized in that the** patient is a person living with asthma, chronic obstructive pulmonary disease, cystic fibrosis, or with another respiratory condition requiring inhaled medication.

13. A system configured to implement the method as defined in any of the preceding Claims, **characterized in that it** comprises:
- An inhalation monitoring device (10) configured for connection to a therapeutic pump (2) and used by a patient during inhalation/exhalation or for inhalation with the said therapeutic pump (2), wherein the inhalation monitoring device (10) comprises:
- a casing (17) configured for connection to the said therapeutic pump (2);
- a power supply unit (18) configured to power the inhalation monitoring device (10);
- a first computing unit (11) of the inhalation monitoring device (10);
- a data storage unit (12) configured to store data collected and/or processed by the inhalation monitoring device (10);
- a set of sensors (14) comprising one or more differential pressure sensors, configured to measure the differential airflow;
- at least one capacitive button (19) configured to activate the inhalation monitoring device (10);
- a communication unit (13) set up to communicate through wired or wireless means with a second computing unit (20);
- a connecting element (17A) of the inhalation monitoring device (10), wherein the connecting element is arrayed in the casing (17);
- at least one light source (16) arrayed in the casing (17) and configured to emit light in at least two distinct colors, a first color and a second color;
- second computing unit (20), wherein the second computing unit (20) comprises:
- a user interface (21) configured to display information to the user, such as being configured to display instructions for an inhalation tutorial and receive user input, and for user data entry, wherein the user may be either the patient (1) or a patient (1) caregiver
wherein the second computing unit (20) is selected from the group comprised of: laptop, desktop computer, tablet, smartphone, smartwatch; and
wherein the second computing unit (20) is set up to communicate with the communication unit (13) of the inhalation monitoring device (10) and with a third computing unit (30), by wired or wireless means of communication;
- third computing unit (30), wherein the third computing unit (30) is a device selected from the group comprised of: server, local network computer, remote cloud computer, laptop, desktop computer, tablet, smartphone or any other computing device accessible through a communications network such as the Internet, wherein the third computing unit (30) is set up to communicate with the second computing unit (20) by wired or wireless means.

14. The system, according to the previous Claim, **characterized in that the** inhalation monitoring device (10) additionally comprises:
- a set of motion sensors (15) comprising one or more vibration and motion sensors configured to measure the position of the inhalation monitoring device (10) when connected to the therapeutic pump (2),
wherein the data captured by the sensor array (15) are sent in Step j) from the communication unit (13) of the inhalation monitoring device (10) to the second computing unit (20) and are then sent in Step k) from the second computing unit (20) to the third computing unit (30).

15. A computer program product **characterized in that** it comprises a set of sequential and logic instructions whereby, when the program is run on a computer, the computer performs the steps of the method as defined in any of Claims 1 through 12.

16. A computer-readable storage medium **characterized in that** it comprises the installation of a computer program product as defined in Claim 15.
